# FASCICULE DE BREVET EUROPEEN

(11) **EP 1 144 906 B1**
(45) Date de publication et mention de la délivrance du brevet: **12.09.2007**
(21) Numéro de dépôt: 00906462.7
(22) Date de dépôt: 23.02.2000
(51) Int. Cl.: F17C 9/02

(54) **DISPOSITIF TRANSPORTABLE DE STOCKAGE ET DE FOURNITURE DE FLUIDE CRYOGENIQUE, PLUS PARTICULIEREMENT D'OXYGENE MEDICAL**
TRAGBARES GERÄT FÜR DIE LAGERUNG UND LIEFERUNG EINES KRYOGENEN FLUIDUMS INSBESONDERE EINES MEDIZINISCHEN SAUERSTOFFES
TRANSPORTABLE DEVICE FOR STORING AND SUPPLYING CRYOGENIC FLUID, ESPECIALLY MEDICAL OXYGEN

(30) Priorité: 29.10.1999 FR 9913617; 23.11.1999 FR 9914730
(43) Date de publication de la demande: 17.10.2001
(73) Titulaire: L'AIR LIQUIDE, Société Anonyme pour l'Etude et l'Exploitation des Procédés Georges Claude, 75007 Paris (FR)
(72) Inventeur: BOUCHER, Guillaume, F-94130 Nogent sur Marne (FR); BERTARIONE, Hervé, F-83136 Meounes les Montrieux (FR)
(74) Mandataire: Le Moenner, Gabriel
(86) Numéro de dépôt international: PCT/FR2000/000449
(87) Numéro de publication internationale: WO 2001/031254

(56) Documents cités:
- GB-A- 191 405 041
- US-A- 3 199 303
- US-A- 4 211 086

## Description

La présente invention concerne les dispositifs transportables de stockage et de fourniture de fluide cryogénique, du type comprenant un réservoir de stockage du fluide sous forme liquide, un circuit de fourniture du fluide sous forme gazeux à au moins un poste utilisateur, agencé sur partie supérieure du réservoir et comprenant au moins un serpentin de vaporisation, et des moyens de manutention du dispositif.

L'invention concerne notamment les dispositifs de fourniture par vaporisation d'un liquide cryogénique, plus particulièrement les dispositifs atmosphériques de vaporisation et de réchauffe d'un liquide cryogénique contenant de l'oxygène pour l'oxygénothérapie, du type comprenant un serpentin constitué plusieurs spires coaxiales d'axe normalement sensiblement vertical, ayant première extrémité raccordée à une source de liquide cryogénique et deuxième extrémité raccordable à un circuit utilisateur, par exemple d'alimentation d'un masque respiratoire.

Dans les dispositifs connus de ce type, tels que décrits dans le document US-A-4211086, le serpentin comporte des spires superposées de diamètre sensiblement constant et d'écartement axial également sensiblement constant. En utilisation, la condensation d'eau qui se forme inévitablement le long du serpentin, se traduit par la formation de gouttes d'eau tombant vers le bas du serpentin, c'est-à-dire sur les spires les plus froides, en s'accumulant sur ces spires inférieures sous forme de couches de glace qui abaissent considérablement les performances d'échange du serpentin et obligent, en pratique, à en augmenter la longueur développée, donc à en augmenter le volume hors tout. Ce phénomène de condensation et de dépôt de glace encore accru dans le cas, le plus fréquent, où le serpentin est abrité dans un couvercle coiffant le réservoir de gaz cryogénique constituant ladite source.

Dans le document US-A-4211086 précité, le portage et la manutention du dispositif sont assurés par des poignées articulées fixées sur parois latérales du réservoir, ce qui ne permet pas une manipulation correcte ni aisée par un utilisateur. De plus, le circuit de fourniture du fluide sous forme gazeuse est enclos dans un boîtier fermé constitué de l'assemblage étanche d'un couvercle sensiblement dépourvu d'ouvertures et d'une embase à profil en T soudée sur le réservoir. Un tel agencement ne permet pas des échanges de chaleur convenables ni l'évacuation de la vapeur d'eau s'accumulant dans le couvercle, notamment du fait du serpentin froid de vaporisation.

On a proposé plus récemment des dispositifs analogues avec une embase support de couvercle profilée reliée par un tube faisant saillie vers l'extérieur du couvercle à une petite bouteille suspendue latéralement au réservoir et recueillant les condensats s'accumulant dans l'embase, dans un agencement mal commode et peu optimisé.

On a aussi proposé dans le document US-A-3199303 considéré comme divulguant l'art antérieur le plus proche, un dispositif aisément transportable de stockage et de fourniture de fluide cryogénique, comprenant :
- un réservoir de stockage de fluides sous forme liquide,
- un circuit de fourniture du fluide sous forme gazeux à au moins un poste utilisateur, agencé sur la partie supérieure du réservoir, dont au moins une partie est abritée dans un couvercle et comprenant au moins un serpentin de vaporisation,
- des moyens de collecte et d'évacuation de condensat pourvus d'un orifice d'évacuation du condensat, qui est ici fermé par une vis de purge.

Toutefois, ce document ne propose aucune solution au problème de la purge des condensats accumulés qui nécessite des interventions régulières avec un matériel spécial, notamment pour dévisser la vis de purge et recueillir les condensats.

La présente invention a pour objet de proposer un dispositif perfectionné permettant, de façon simple, efficace, à faibles coûts offrant une grande versatilité, de recueillir et éliminer avec une grande efficacité les condensats dans le couvercle.

Dans ce but, l'invention propose un dispositif du type de celui décrit et représenté dans le document US-A-3199303, caractérisé en ce que le couvercle comporte des moyens d'accrochage d'un récipient de recueil de condensat amovible.

D'autres caractéristiques de l'invention sont définies aux revendications secondaires.

D'autres caractéristiques des modes de réalisation et avantages de l'invention ressortiront la description suivante d'un mode de réalisation, donnée à titre illustratif mais nullement limitatif faite en relation avec le dessin annexé, sur lequel:

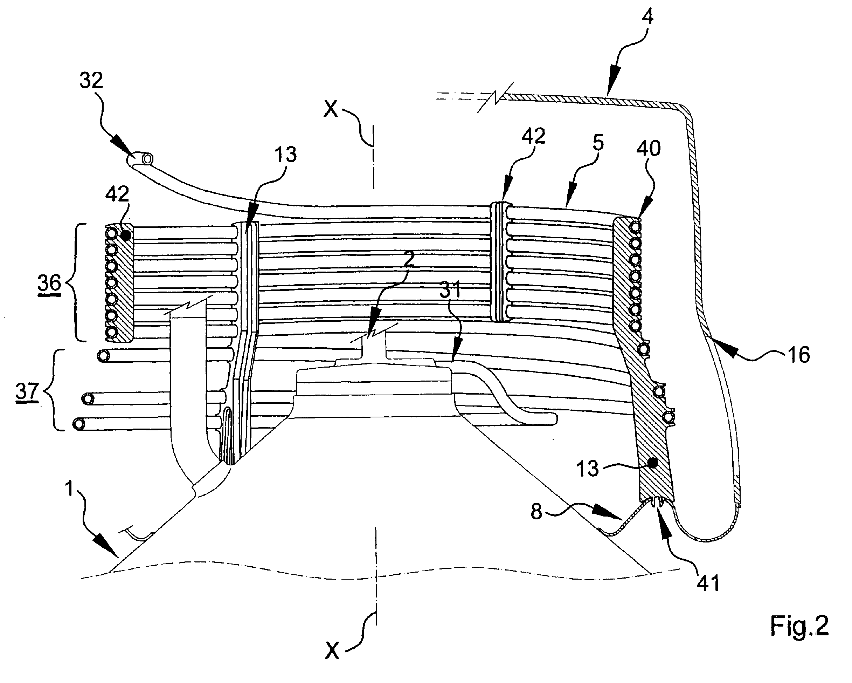

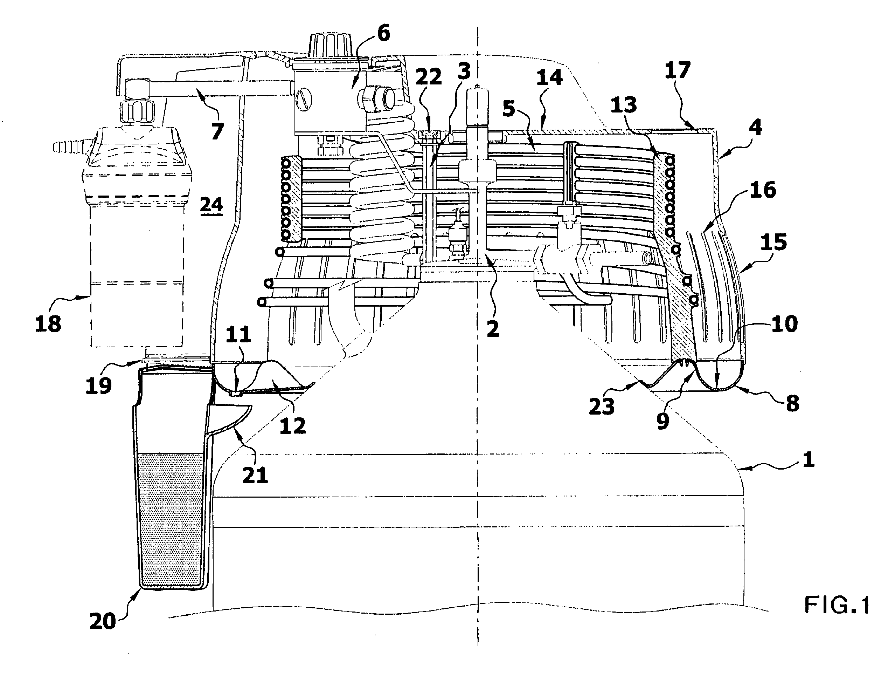

- la figure 1 représente schématiquement, partiellement en coupe, la partie supérieure d'un dispositif selon l'invention.
- la figure 2 représente, à plus grande échelle, un serpentin de vaporisation.

Sur les figures, on reconnaît la partie supérieure, conique, d'un réservoir liquide cryogénique sous pression 1, typiquement d'oxygène liquide, d'axe vertical.

A son extrémité supérieure, le réservoir comporte une tête 2 remplissage et d'extraction de gaz comportant notamment une tige verticale de fixation, avec une vis 22, d'un couvercle 4 ou capot, comme on le verra plus avant. A la tête 2 est raccordée l'extrémité amont d'un serpentin vaporisation 5, avantageusement coaxial au réservoir 1 et dont l'extrémité aval est reliée, via un bloc robinet/détendeur 6, à une sortie de gaz 7 de connexion à un circuit utilisateur, typiquement un circuit de ventilation d'un patient (non représenté). Avantageusement, au moins la base du serpentin 5 comporte des spires plus écartées axialement les unes des autres que les spires supérieures et de diamètres croissant vers le bas pour empêcher le givrage de gouttes d'eau de condensation apparaissant sur les spires les moins froides serpentin et gouttant vers le bas par gravité.

Ces gouttes de condensation sont recueillies dans une structure de collerette annulaire, généralement désignée par la référence 8, soudée par sa partie centrale sur la paroi conique 23 du réservoir 1 et présentant, en coupe transversale, à partir de cette partie centrale un profil formant successivement une zone bombée vers le haut puis une zone bombée vers le bas périphérique 10 formant une main courante de préhension circulaire, le creux de la partie bombée 9 permettant de loger doigts la main du manutentionnaire. En un point de la main courante il est prévu, dans le fond de la partie bombée périphérique 10, un orifice d'égouttage 11 permettant d'évacuer par gravité l'eau condensée recueillie dans collerette 8. L'orifice 11 est avantageusement formé dans le point le plus de la collerette 8 qui présente, à cet effet, une légère déclivité dans la direction vers l'orifice 11. Une partie substantielle des condensats gouttant dans partie centrale de la collerette 8, il est prévu, au moins au niveau de l'orifice 1 au moins une vallée radiale 12 interrompant localement la partie bombée intermédiaire 9 pour permettre une libre communication de liquide de part d'autre de cette dernière.

Dans un mode de réalisation préféré, comme représenté, le serpentin est supporté par au moins un peigne support 13 en matériau faiblement conducteur de la chaleur, typiquement en matériau plastique, dont la partie basse est montée, avantageusement par simple clipsage, sur la partie bombée intermédiaire 9, pourvue typiquement, en partie haute, d'orifices à cet effet.

Le couvercle 4 comporte une paroi transversale supérieure 14 servant à la fixation du couvercle 4 sur le réservoir 1 via un pilier vertical 3, et une partie périphérique en forme de jupe évasée vers bas 15 dont l'arête inférieure vient porter en appui sur l'arête périphérique de la partie bombée 10 de la collerette 8. Pour réaliser une vaporisation atmosphérique avec des échanges thermiques optimisés, la jupe 15 comporte, typiquement sur l'essentiel de son pourtour, une série de fentes verticales parallèles 16 et la paroi supérieure 14 comporte, au voisinage de périphérie, une série de fentes radiales 17.

Le couvercle 4 forme latéralement un boîtier définissant un logement 24 pour recevoir au moins en partie un récipient humidificateur indiqué en traits pointillés 18 venant se raccorder à canalisation de sortie 7. Avantageusement, le logement 24 comporte à sa base des moyens 19 d'accrochage démontable, avantageusement du type a glissière, d'un récipient récepteur de condensat 20 comportant une goulotte 21 s'étendant, en position accrochée du récipient 20, au-dessous de l'orifice d'évacuation 11.

Le couvercle 4 et le récipient 20 sont avantageusement réalisés matériaux plastiques rigides, Le réservoir 1 comporte avantageusement, à sa base, des roulettes facilitant son déplacement.

Comme on le voit mieux sur la Figure 2, le serpentin 5, constitué par bobinage d'un tube métallique bon conducteur de la chaleur, classiquement en aluminium, sur un mandrin profilé, est divisé en une partie supérieure 36 comportant au moins deux, typiquement six ou sept spires adjacentes et d'un diamètre sensiblement constant, et une partie inférieure 37, entourant partiellement l'extrémité supérieure du réservoir 1, comprenant au moins deux, en pratique trois ou quatre spires, plus écartées axialement les unes des autres et de diamètres croissant vers le bas, la spire complète la plus basse ayant le plus grand diamètre et la spire complète la plus haute ayant un diamètre légèrement supérieur à celui des spires de la partie supérieure 36.

De cette façon, les gouttes d'eau de condensation apparaissant sur les spires les moins froides de la partie supérieure 36 s'écoulent et s'égouttent vers le bas sans tomber, ou très peu, sur les spires les plus froides de la partie inférieure 37, pour s'accumuler dans une gouttière 8 d'où l'eau est évacuée vers le récipient recueil de condensats 20. De plus, l'écartement axial et radial des spires inférieures garantit un échange thermique grandement amélioré avec l'atmosphère environnante.

Avantageusement, comme sus-mentionné, le serpentin 5 est supporté par des peignes supports 13 angulairement séparés, conformés pour correspondre au profil du serpentin 5 et comportant extérieurement une série de logements 40 agencés pour recevoir les différentes spires du serpentin 5. Les peignes supports 13, en matériau faiblement conducteur de la chaleur, typiquement en matériau plastique, sont avantageusement montés à leur base par clipsage 41 dans la structure formant gouttière 8. Avantageusement, les spires supérieures 36 du serpentin 5 sont maintenues en position, en sus des peignes supports 13, par des peignes entretoises 42 de hauteur réduite.

## Revendications

1. Dispositif transportable de stockage et de fourniture de fluide cryogénique, comprenant :
- un réservoir (1) de stockage de fluides sous forme liquide,
- un circuit de fourniture du fluide sous forme gazeux à au moins un poste utilisateur, agencé sur la partie supérieure du réservoir, dont au moins une partie est abritée dans un couvercle (4) et comprenant au moins un serpentin de vaporisation (5),
- des moyens (8) de collecte et d'évacuation de condensat pourvus d'un orifice (11) d'évacuation du condensat,
**caractérisé en ce que** le couvercle (4) comporte des moyens (19) d'accrochage d'un récipient (20) de recueil de condensat amovible:

2. Dispositif selon la revendication 1, **caractérisé en ce que** le couvercle (4) comporte des moyens (19) d'accrochage démontable du type a glissière, du récipient récepteur de condensat (20).

3. Dispositif selon l'une quelconque des revendications précédentes, **caractérisé en ce que** lesdits moyens de collecte et d'évacuation de condensat comportent une collerette profilée (8) montée sur le réservoir (1), formant gouttière de collecte et d'évacuation de condensat et pourvue de l'orifice (11) d'évacuation du condensat.

4. Dispositif selon la revendication précédente, **caractérisé en ce que** des moyens de manutention du dispositif sont au moins en partie constitués par ladite collerette profilée (8) montée sur le réservoir (1).

5. Dispositif selon la revendication 4, **caractérisé en ce que** la collerette (8) comprend une partie extérieure (10) bombée vers le bas formant main courante se raccordant à une partie centrale de montage de la collerette sur le réservoir par une partie médiane (9) bombée vers le haut et pourvue d'au moins un passage radial en creux (12).

6. Dispositif selon la revendication 5, **caractérisé en ce que** le serpentin (5) est supporté par des peignes supports (13) montés sur la partie médiane (9) de la collerette (8).

7. Dispositif selon l'une quelconque des revendications précédentes, **caractérisé en ce que** le couvercle comporte une partie d'extrémité inférieure en forme de jupe évasée (15) venant porter en appui sur la périphérie de la collerette (8).

8. Dispositif selon la revendication 7, **caractérisé en ce que** le couvercle (4) comporte des ouvertures d'aération (16) angulairement réparties sur la jupe (15).

9. Dispositif selon l'une quelconque des revendications 4 à 6, **caractérisé en ce que** le récipient (20) comporte une goulotte (21) de réception de condensat s'étendant, en configuration accrochée, sous l'orifice (11) d'évacuation de la collerette (8).

10. Dispositif selon l'une quelconque des revendications précédentes, **caractérisé en ce que** le couvercle (4) comprend un logement latéral (24) pour recevoir au moins en partie un récipient humidificateur (18) insérable en sortie du circuit de fourniture du fluide.

11. Dispositif selon l'une des revendications 1 à 10, **caractérisé en ce que** le serpentin (5) comporte des spires de diamètres différents.

12. Dispositif selon la revendication précédente, **caractérisé en ce que** le serpentin (5) est constitué de plusieurs spires coaxiales d'axe sensiblement vertical (X), et **en ce qu'**au moins les deux spires inférieures du serpentin (5) ont un plus grand diamètre que les autres spires.

13. Dispositif selon la revendication 12, **caractérisé en ce qu'**au moins les deux spires supérieures du serpentin (5) ont un même diamètre réduit.

14. Dispositif selon la revendication 12 ou la revendication 13, **caractérisé en ce qu'**au moins les deux spires inférieures ont un diamètre diminuant vers le haut.

15. Dispositif selon la revendication 14, **caractérisé en ce que** la distance axiale moyenne interspires est plus grande pour les spires inférieures (37) que pour les spires supérieures (36).

16. Dispositif selon l'une des revendications 12 à 15, **caractérisé en ce que** le serpentin (5) est disposé dans une enceinte non hermétique formant un couvercle pour la partie supérieure du réservoir (1).

17. Dispositif selon l'une des revendications précédentes, **caractérisé en ce que** le fluide cryogénique est de l'oxygène.

## Claims

1. Transportable device for storing and supplying cryogenic fluid, comprising:
- a tank (1) for storing fluids in liquid form,
- a circuit for supplying the fluid in gaseous form to at least one user station, arranged on the upper part of the tank, whereof at least part is sheltered in a lid (4) and comprising at least one vaporization coil (5),
- means (8) for collecting and removing condensate provided with a condensate removal orifice (11),
**characterized in that** the lid (4) comprises means (19) for fastening a receptacle (20) for collecting removable condensate.

2. Device according to Claim 1, **characterized in that in that** the lid (4) comprises removable means (19) of the slide type, for fastening the condensate receiving receptacle (20).

3. Device according to either of the preceding claims, **characterized in that** the said condensate collection and removal means comprise a profiled flange (8) mounted on the tank (1), forming a condensate collection and removal channel and provided with the condensate removal orifice (11).

4. Device according to the preceding claim, **characterized in that** means for handling the device consist at least partly of the said profiled flange (8) mounted on the tank (1).

5. Device according to Claim 4, **characterized in that** the flange (8) comprises a downwardly convex outer part (10) forming a railing and connected to a central part for assembling the flange on the tank via an upwardly convex median part (9) and provided with at least one hollow radial passage (12).

6. Device according to Claim 5, **characterized in that** the coil (5) is supported by support combs (13) mounted on the median part (9) of the flange (8).

7. Device according to any one of the preceding claims, **characterized in that** the lid comprises a lower end part in the form of a flared skirt (15) supporting the periphery of the flange (8).

8. Device according to Claim 7, **characterized in that** the lid (4) comprises aeration openings (16) angularly distributed on the flange (15).

9. Device according to any one of Claims 4 to 6, **characterized in that** the receptacle (20) comprises a condensate receiving chute (21) extending, in the fastened configuration, under the removal orifice (11) of the flange (8).

10. Device according to any one of the preceding claims, **characterized in that** the lid (4) comprises a lateral recess (24) for at least partly receiving a humidifier receptacle (18) insertable at the outlet of the fluid supply circuit.

11. Device according to one of Claims 1 to 10, **characterized in that** the coil (5) comprises turns of different diameters.

12. Device according to the preceding claim, **characterized in that** the coil (5) consists of a plurality of coaxial turns having a substantially vertical axis (X), and **in that** at least the two lower turns of the coil (5) have a larger diameter than the other turns.

13. Device according to Claim 12, **characterized in that** at least the upper two turns of the coil (5) have one and the same reduced diameter.

14. Device according to either of Claims 12 and 13, **characterized in that** at least the lower two turns have a diameter decreasing upwards.

15. Device according to Claim 14, **characterized in that** the average axial inter-turn distance is larger for the lower turns (37) than for the upper turns (36).

16. Device according to one of Claims 12 to 15, **characterized in that** the coil (5) is placed in a non-hermetically sealed chamber forming a lid for the upper part of the tank (1).

17. Device according to one of the preceding claims, **characterized in that** the cryogenic fluid is oxygen.

## Patentansprüche

1. Tragbare Lager- und Versorgungsvorrichtung für kryogenes Fluid, umfassend:
- einen Lagertank (1) für Fluide in flüssiger Form,
- einen Versorgungskreislauf für das gasförmige Fluid für mindestens eine Verbrauchsstelle, die an dem oberen Teil des Tanks angeordnet ist, von der mindestens ein Teil in einem Deckel (4) untergebracht ist und die mindestens eine Verdampferschlange (5) umfasst,
- Mittel (8) zum Sammeln und zum Ablassen von Kondensat, die mit einer Öffnung (11) zum Ablassen des Kondensats versehen sind,
**dadurch gekennzeichnet, dass** der Deckel (4) Mittel (19) zum Einhängen eines abnehmbaren Auffangbehälters (20) von Kondensat aufweist.

2. Vorrichtung nach Anspruch 1, **dadurch gekennzeichnet, dass** der Deckel (4) schienenartige Mittel (19) zum lösbaren Einhängen des Aufnahmebehälters von Kondensat (20) aufweist.

3. Vorrichtung nach irgendeinem der vorhergehenden Ansprüche, **dadurch gekennzeichnet, dass** die Mittel zum Sammeln und zum Ablassen von Kondensat einen profilierten Kragen (8) aufweisen, der an dem Tank (1) angebracht ist und eine Rinne zum Sammeln und Ablassen von Kondensat ausbildet und mit der Öffnung (11) zum Ablassen des Kondensats versehen ist.

4. Vorrichtung nach dem vorhergehenden Anspruch, **dadurch gekennzeichnet, dass** die Beförderungsmittel der Vorrichtung mindestens teilweise aus dem profilierten Kragen (8) bestehen, der an dem Tank (1) angebracht ist.

5. Vorrichtung nach Anspruch 4, **dadurch gekennzeichnet, dass** der Kragen (8) einen Außenteil (10) umfasst, der nach unten gewölbt ist und einen Handlauf ausbildet, der an einen zentralen Montageteil des Kragens an dem Tank durch einen mittleren Teil (9) angeschlossen ist, welcher nach oben gewölbt ist und mit mindestens einem radialen hohlen Durchgang (12) versehen ist.

6. Vorrichtung nach Anspruch 5, **dadurch gekennzeichnet, dass** die Schlange (5) durch Haltekämme (13) gehalten wird, die an dem mittleren Teil (9) des Kragens (8) angebracht sind.

7. Vorrichtung nach irgendeinem der vorhergehenden Ansprüche, **dadurch gekennzeichnet, dass** der Deckel einen unteren Endteil in Form einer konisch erweiterten Schürze (15) aufweist, die sich an dem Umfang des Kragens (8) abstützt.

8. Vorrichtung nach Anspruch 7, **dadurch gekennzeichnet, dass** der Deckel (4) Belüftungsöffnungen (16) aufweist, die winkelförmig an der Schürze (15) verteilt sind.

9. Vorrichtung nach irgendeinem der Ansprüche 4 bis 6, **dadurch gekennzeichnet, dass** der Behälter (20) eine Auffangrinne (21) zum Aufnehmen von Kondensat aufweist, die sich, in der eingehängten Ausgestaltung, unter der Öffnung (11) zum Ablassen des Kragens (8) befindet.

10. Vorrichtung nach irgendeinem der vorhergehenden Ansprüche, **dadurch gekennzeichnet, dass** der Deckel (4) eine seitliche Aufnahme (24) umfasst, um mindestens teilweise einen Luftbefeuchtungsbehälter (18) aufzunehmen, der am Ausgang des Versorgungskreislaufs mit Fluid einsetzbar ist.

11. Vorrichtung nach einem der Ansprüche 1 bis 10, **dadurch gekennzeichnet, dass** die Schlange (5) Windungen mit unterschiedlichen Durchmessern aufweist.

12. Vorrichtung nach dem vorhergehenden Anspruch, **dadurch gekennzeichnet, dass** die Schlange (5) aus mehreren koaxialen Windungen mit im Wesentlichen vertikaler Achse (X) besteht und dass mindestens die beiden unteren Windungen der Schlange (5) einen größeren Durchmesser als die anderen Windungen haben.

13. Vorrichtung nach Anspruch 12, **dadurch gekennzeichnet, dass** mindestens die beiden oberen Windungen der Schlange (5) denselben reduzierten Durchmesser haben.

14. Vorrichtung nach Anspruch 12 oder nach Anspruch 13, **dadurch gekennzeichnet, dass** mindestens die beiden unteren Windungen einen nach oben abnehmenden Durchmesser haben.

15. Vorrichtung nach Anspruch 14, **dadurch gekennzeichnet, dass** der mittlere axiale Abstand zwischen den Windungen bei den unteren Windungen (37) größer ist als bei den oberen Windungen (36).

16. Vorrichtung nach einem der Ansprüche 12 bis 15, **dadurch gekennzeichnet, dass** die Schlange (5) in einer nicht hermetischen Umfassung angeordnet ist, die einen Deckel für den oberen Abschnitt des Tanks (1) ausbildet.

17. Vorrichtung nach einem der vorhergehenden Ansprüche, **dadurch gekennzeichnet, dass** das kryogene Fluid Sauerstoff ist.
